# EUROPÄISCHE PATENTSCHRIFT

(11) **EP 2 792 379 B1**
(45) Veröffentlichungstag und Bekanntmachung des Hinweises auf die Patenterteilung: **13.04.2016**
(21) Anmeldenummer: 13001970.6
(22) Anmeldetag: 15.04.2013
(51) Int. Cl.: A61M 5/31, A61L 31/08, B05D 1/00, B05D 7/00, B65D 83/00, C08J 7/04, C23C 16/00, A61M 39/08, A61B 5/15

(54) **Hydrophil innenbeschichtetes medizintechnisches Gerät**
Hydrophilically internally coated medical device
Appareil médical doté d'un revêtement intérieur hydrophile

(43) Veröffentlichungstag der Anmeldung: 22.10.2014
(73) Patentinhaber: Genigo GmbH, 22337 Hamburg (DE)
(72) Erfinder: Görne, Martin, 22337 Hamburg (DE)
(74) Vertreter: Qip Patentanwälte Dr. Kuehn & Partner mbB

(56) Entgegenhaltungen:
- US-A1- 2003 031 806
- US-A1- 2010 132 762
- US-A1- 2012 123 345

## Beschreibung

Die vorliegende Erfindung bezieht sich auf ein hydrophil innenbeschichtetes medizintechnisches Gerät zur temporären Aufnahme einer Körperflüssigkeit, insbesondere Blut, oder einer Zellsuspension darin. Insbesondere kann das Gerät eine Flasche, ein Beutel, ein Rohr oder ein Schlauch sein, aber auch ein aktivierbarer Teil einer Vorrichtung zur Beaufschlagung der Körperflüssigkeit bzw. des Blutes bzw. der Zellsuspension mit einer äußeren Einwirkung.

Vorrichtungen zur temporären Aufnahme von Blut sind bekannt, sowohl solche zum Hindurchleiten wie auch solche zum zeitweisen Speichern. Beispielhaft sei der in der deutschen Offenlegungsschrift DE 10 2011 108 787 A1 beschriebene Blutschlauch erwähnt, welche Offenlegungsschrift hiermit, insoweit als die Ausgestaltung eines Blutführungssystems betroffen ist, durch Bezugnahme eingeschlossen wird.

Ein grundsätzliches Phänomen ist, dass sich die Gerinnungsneigung des Blutes oder gewisser Eiweißbestandteile in der Körperflüssigkeit durch den Kontakt mit der Vorrichtung regelmäßig erhöht. Dies ist meist unerwünscht, und dem wird nötigenfalls durch die Zugabe gerinnungshemmender Mittel entgegengewirkt. Andererseits haben gerinnungshemmende Mittel an sich unerwünschte Wirkungen, sei es, dass eine Blutkonserve im Zuge einer Operation zum Einsatz kommt, und der Operateur an sich Blutungen schnell gestillt haben möchte; sei es, dass eine Körperflüssigkeit möglichst rein und unkontaminiert rückgeführt oder weiterverarbeitet werden soll. Ähnliche Probleme betreffen Zellsuspensionen, die einem Wirbel(Säuge-)tier oder einer Person zugeführt werden sollen.

Es ist bereits aus der Patentanmeldung US 2012/0123345 A1 bekannt, Innenoberflächen von Gefäßen zu beschichten, indem eine Elektrode in den Hohlraum des Gefäßes eingeführt und in Gegenwart eines Reaktivgases das Plasma gezündet wird. Diese (hohle) Elektrode dient gleichzeitig als Gaszuführung, die (konkave) Gegenelektrode wird um das Gefäß herum angeordnet.

In Hinblick auf die Anordnung der Gerätschaften wird die Offenbarung dieser Patentanmeldung wiederum durch Bezugnahme eingeschlossen. Die so erhaltenen innenbeschichteten Gefäße sind allerdings für die Zwecke der temporären Aufnahme von Körperflüssigkeiten, Blut bzw. Zellsuspensionen noch nicht zufriedenstellend.

Die vorliegende Erfindung setzt sich zum Ziel, die Nachteile der bekannten Vorrichtungen und Verfahren zu vermindern oder zu beseitigen.

Dazu schlägt die Erfindung einen innenbeschichteten Hohlkörper gemäß Anspruch 1, und ein Verfahren zur Innenbeschichtung gemäß Anspruch 11 vor.

Unter einem ersten Aspekt weist ein erfindungsgemäßer Hohlkörper an seiner inneren Oberfläche eine hydrophilierende Beschichtung auf. Dadurch wird die Gerinnungsneigung der darin aufgenommenen oder dahindurch geleiteten Körperflüssigkeit bzw. des Blutes bzw. der Zellsuspension weniger erhöht, als dies bisher möglich war. Insbesondere wird ein Wasserkontaktwinkel, welche Größe die Hydrophilie einer Oberfläche beschreibt, von unter 2° oder sogar unter 0,5° eingestellt. In Ausführungsformen ist das Oberflächenmaterial des Hohlkörpers ein Kunstharz, und ist die hydrophilierende Beschichtung aus Acrylsäureeinheiten aufgebaut. Der Hohlkörper kann genau eine Öffnung aufweisen, und eine Flasche oder ein Beutel sein; er kann aber auch zwei Öffnungen aufweisen, durch welche bei Benutzung das Blut bzw. die Körperflüssigkeit bzw. die Zellsuspension strömt, und dabei entweder ein Rohr oder Schlauch sein, oder auch ein aktivierbarer Teil einer die Körperflüssigkeit bzw. die Zellsuspension bei ihrem Betrieb mit einer äußeren Einwirkung beaufschlagenden Vorrichtung sein.

Unter einem zweiten Aspekt weist ein erfindungsgemäßes Verfahren einen Aktivierungsschritt, einen Vorbeschichtungsschritt und einen Nachbeschichtungsschritt auf. Dabei ist im Aktivierungsschritt hauptsächlich nur Inertgas vorhanden, im Vorbeschichtungsschritt sowohl Inertgas als auch ein Reaktivgas, und im abschließenden Nachbeschichtungsschritt hauptsächlich nur das Reaktivgas, und zwar ohne Plasmaeinwirkung. In Ausführungsformen dauert die Vorbeschichtungsphase unter einer Minute; das Oberflächenmaterial des Hohlkörpers kann wie oben angegeben ein Kunstharz sein, das Beschichtungsmaterial ist in Ausführungsformen Acrylsäure oder deren Anhydrid.

Er wird vermutet, dass die besonders kurze Dauer der Vorbeschichtungsphase es ermöglicht, dass aktivierte Stellen der Oberfläche nicht sogleich wieder deaktiviert werden, so dass die zweischrittige PECVD-/CVD-Beschichtung besonders effektiv zu einer hydrophilen Oberfläche führt.

Weitere Merkmale der Erfindung ergeben sich aus der nachfolgenden Beschreibung von Ausführungsbeispielen in Verbindung mit den Figuren. In diesen werden gleiche oder ähnliche Elemente mit gleichen bzw. ähnlichen Bezugszeichen bezeichnet. Die Erfindung ist nicht auf die beschriebenen Ausführungsbeispiele beschränkt, sondern durch den Umfang der beiliegenden Patentansprüche bestimmt. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung von Ausführungsbeispielen der Erfindung wird auf die beiliegenden Figuren Bezug genommen, von denen
- Figur 1: einen schematischen Querschnitt durch einen Hohlkörper gemäß der Erfindung und
- Figur 2: ein Flussdiagrammschema eines erfindungsgemäßen Verfahrens zeigt.

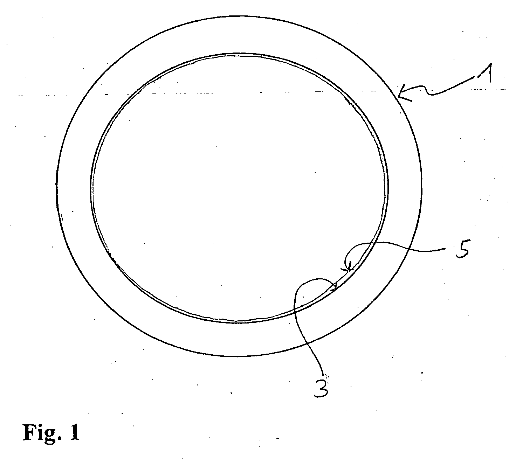

Die Figur 1 zeigt einen allgemein ovalen, in der gezeigten Ausführungsform im Wesentlichen kreisrunden Querschnitt des Hohlkörpers 1. Obwohl die Wandung als einheitlich und nahtlos dargestellt ist, kann sie mehrschichtig sein und/oder eine oder mehrere Nähte aufweisen. Die innere Oberflächenschicht 3 ist aus einem Kunstharz gebildet, wobei darunter vernetzte, aber auch unvernetzte polymere Materialien verstanden werden sollen, letzteres insbesondere bei zweischichtigen Wandungen, bei denen die mechanische Stabilität überwiegend von der oder den äußeren Schicht/en bereitgestellt wird. Der Wasserkontaktwinkel der inneren Oberflächenschicht beträgt 10° oder mehr, insbesondere 30° oder mehr, und die Oberflächenschicht wird in diesem Zusammenhang als wenig hydrophil bezeichnet. Durch das weiter unten beschriebene kombinierte PECVD/CVD-Beschichtungsverfahren mit ultrakurzer Vorbeschichtung wird auf der inneren Oberfläche eine sehr dünne Haftschicht und darauf eine dickere Funktionsschicht 5 ausgebildet, beide aus carboxygruppenhaltigen, z. B. Acrylsäure-Einheiten. Diese Schichten sind dauerhaft mit der inneren Oberflächenschicht verbunden und überraschenderweise äußerst hydrophil, und zwar hydrophiler als solche, die durch ansonsten gleiche Weise, aber mit längerer (> 1 Minute) Vorbeschichtungsphase erhalten werden. Es wird vermutet, dass eine längere Vorbeschichtung die einmal erzeugten aktiven Stellen weiter- oder miteinander reagieren lässt, und zwar in der erzielbaren Hydrophilie abträglicher Weise. Gleichwohl ist eine gewisse Einwirkungszeit des Plasmas, von z. B. mindestens 2 Sekunden, durchaus erforderlich, um eine stabile Beschichtung zu ermöglichen.

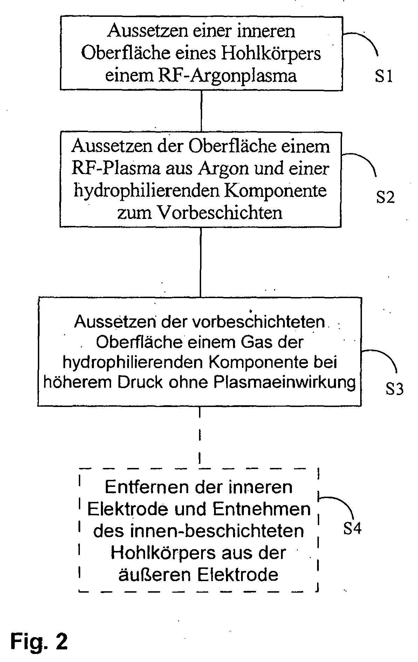

Das Flussdiagrammschema der Figur 2 veranschaulicht die wesentlichen Schritte eines Verfahrens zum Hydrophilieren von Innenoberflächen durch Beschichten mit Polyacrylsäure: Nach dem Anordnen einer Hohlelektrode in dem Gefäß bzw. dem einseitig verschlossenen Rohr oder Schlauch wird jenes bzw. jener mittels Pumpen evakuiert, vorzugsweise auf einen Druck von maximal 10⁻⁴ mbar (10 mPa). Nach Erreichen des gewünschten Vakuumdrucks wird das Gefäßinnere bei dauerndem Pumpen mit einem Inertgas, vorzugsweise Argon, geflutet, wobei der Inertgaszufluss so auf die Pumpleistung abgestimmt wird, dass sich im Innenraum des Gefäßes ein konstanter Gasdruck ausbildet. Das Inertgas wird aus einer Inertgasquelle zugeführt. Bei bevorzugten Ausführungsformen beträgt der so eingestellte Argongasdruck etwa 25-150 mTorr (3,33-20 Pa). Nach Erreichen eines stabilen Inertgasdrucks im Gefäßinneren wird der Plasmagenerator, beispielsweise ein Hochfrequenzgenerator, eingeschaltet, um ein Inertgasplasma zu schaffen. Das Plasma reinigt die Innenoberflächen durch Entfernen von daran adsorbierten Stoffen und resultiert ferner in einer Aktivierung der Innenoberflächen durch Bildung von Ionen und freien Radikalen, die den nachfolgenden Aufpolymerisierungsprozess begünstigen.

Die Reinigungs- und Aktivierungswirkung kann in diesem ersten Schritt S1 über die Frequenz des Gasplasmas, die in das Plasma eingespeiste Leistung, die Einwirkzeit des Plasmas und die Art des für das Plasma verwendeten Inertgases beeinflusst werden. Als Inertgas wird bei dem hier vorgestellten Verfahren Argon bevorzugt, da es eine Aktivierung der Innenoberflächen ohne Erzeugen neuer unerwünschter Verbindungen ermöglicht. Selbstverständlich können stattdessen auch andere Inertgase wie N₂ verwendet werden. Bei einer beispielhaften Ausführungsform des Verfahrens beträgt die Einwirkzeit des Argonplasmas 20 bis 120 Sekunden. Nach Ablauf der Einwirkzeit wird der Plasmagenerator ausgeschaltet und das Verfahren mit dem ersten Beschichtungsschritt S2 fortgeführt.

Abweichend vom bisher Dargestellten kann das Plasma statt auf Basis reinen Argons auch auf Basis eines Gemisches von Inertgas und einer im nachfolgenden Vorbeschichtungsprozess verwendeten Reaktivkomponente erzeugt werden. Der Partialdruck der Reaktivkomponente im Gasgemisch sollte aber weniger als ein Zehntel des Partialdrucks des Inertgases betragen.

Beim Übergang von Schritt S1 zu Schritt S2 des Verfahrens wird der Inertgaszufluss ins Gefäßinnere vorzugsweise aufrechterhalten und gegebenenfalls so geändert, dass er einen zur Durchführung von Schritt S2 geeigneten Wert aufweist. Zur Herstellung des Gasgemisches wird dem Inertgas im ein aus carboxygruppenhaltigen Monomeren in der Dampfphase gebildetes erstes Schichtmaterialgas zugemischt. Bei den carboxygruppenhaltigen Monomeren handelt es sich vorzugsweise um Acrylsäure oder einen Acrylsäurevorläufer, wie z. B. Acrylsäureanhydrid. Der Partialdruck P_{eSG} des ersten Schichtmaterialgases beträgt bei Ausführungsformen vorzugsweise mindestens ein Viertel und maximal das Doppelte des Partialdrucks p_{IG} des Inertgases. Besonders bevorzugt ist das Partialdruckverhältnis p_{eSG} : p_{IG} aus einem Bereich von 1:1 bis 1:0,5 ausgewählt. Beispielsweise beträgt der Partialdruck von Argon bei Ausführungsformen des Verfahrens 30 mTorr (ca. 400 mPa) bei einem Gesamtdruck des Gasgemisches von 45 mTorr (ca. 600 mPa), woraus sich ein Wert von 2:1 bezüglich des Verhältnisses von Argonpartialdruck p_{Ar} zu erstem Schichtmaterialgaspartialdruck (Reaktivkomponentenpartialdruck) P_{eSG} ergibt. Während der Einstellung der gewünschten Reaktivgasmischung ist der Plasmagenerator vorübergehend ausgeschaltet.

Als Reaktivkomponente zur Ausbildung des ersten Schichtmaterialgases wird bevorzugt (Meth)Acrylsäureanhydrid verwendet, das in einem gesonderten Behälter verdunstet und dann An den Innenraum des Gefäßes geleitet wird. Der Partialdruck des Schichtmaterialgases wird über dessen Zufluss eingestellt, der mittels Ventilen gesteuert wird. Statt (Meth)Acrylsäureanhydrid kann natürlich auch (Meth)Acrylsäure verwendet werden. (Meth)Acrylsäure bzw. (Meth)Acrylsäureanhydrid werden in Quellbehältern in flüssiger Form, beispielsweise in einer Menge von 150 mL, bereitgestellt. Um ein Polymerisieren der Acrylsäure bzw. deren Vorlaufermaterialien zu verhindern oder zu verzögern, können diese mit Cu(I)-chlorid versetzt werden. Ferner werden die Reaktivkomponentenbehälter nach dem Befüllen solange entlüftet, bis keine Blasen mehr in der Reaktivkomponentenflüssigkeit aufsteigen. Der Dampfdruck der Reaktivkomponenten ist bei den üblichen Raumtemperaturen von 22 bis 25°C zur Bildung des ersten Schichtmaterialgases in der Regel ausreichend.

Nach Einstellen des gewünschten Gasgemisches und Gasgemischdrucks, was typischerweise 1 bis 2 Minuten in Anspruch nimmt, wird der eigentliche Vorbeschichtungsvorgang durch Starten des Plasmagenerators eingeleitet, wodurch im Plasma erzeugte angeregte Acrylsäuremonomere sich an der aktivierten Innenoberfläche anlagern und im weiteren Verlauf dort eine Polyacrylsäureschicht ausbilden. Diese plasmaunterstützte Vorbeschichtungsphase (PECVD) wird nur kurz aufrechterhalten, um eine sehr dünne Haftvermittlerschicht aufzubauen. Die Vorbeschichtungsphase kann unter 60 oder zwischen 2 und 20 Sekunden dauern, was in einem besonders niedrigen Kontaktwinkel für Wasser resultiert. Die Gaszufuhren werden während der Plasmabeschichtung vorzugsweise nicht verändert. Bei der ersten Variante des Verfahrens wird der Vorbeschichtungsprozess durch Abschalten des Plasmagenerators beendet.

Der beschriebenen ersten Variante des Vorbeschichtungsschritts S2 schließt sich eine erste Variante des Folgebeschichtungsschritts S3 an, bei der nach dem Ausschalten des Plasmagenerators zunächst der Inertgaszufluss unterbrochen und die vorbeschichtete Innenoberfläche dem möglichst vollen Dampfdruck einer von wasserfreier Acrylsäure gebildeten Reaktivkomponente ausgesetzt wird. Dieser Dampfdruck sollte ca. 5 Torr (667 Pa) nicht unterschreiten. Leichtes Kühlen oder Erwärmen der Reaktivkomponente im Quellbehälter kann dabei zur Druckeinstellung zweckmäßig sein. Das Einbringen der Reaktivkomponente in das Gefäßinnere bei vollem Dampfdruck stellt Reaktivgas in großen Mengen bereit, das mit den an der vorbeschichteten Oberfläche vorhandenen reaktiven Zentren reagiert und eine vergleichsweise dicke Polyacrylsäureschicht (PAA-Schicht) aufbaut, die kristallin sein kann.

Bei einer zweiten Variante des Verfahrens wird der Plasmagenerator am Ende des Vorbeschichtungsschrittes S2 nicht abgeschaltet und ist daher auch beim Übergang zur zweiten Variante des Folgebeschichtungsschrittes S3 noch in Betrieb. Bei dieser Variante wird die Argonzufuhr fast oder ganz gestoppt und die Zufuhr des Reaktivgases, d. h. der Acrylsäure, so weit reduziert, dass, bei aufrechterhaltener Hochfrequenzerzeugung und fortlaufendem Evakuieren des Gefäßinneren, ein Druckgleichgewicht im Bereich von weniger als 0,3 mTorr (ca. 40 mPa) eingestellt wird. In einer beispielhaften Ausführungsform wird der Druck auf einen Wert von weniger als 0,1 mTorr (ca. 13 mPa) eingestellt. Das Plasma erlischt dabei allmählich aufgrund der Druckerniedrigung.

Die Folgebeschichtungsphase wird in beiden Varianten für 1 bis 45, oder 5 bis 15 Minuten aufrechterhalten.

Bei Beendigung des Verfahrens in Schritt S4 können die innenbeschichteten Gefäße nebst der Hohlelektrode entnommen und kann die Innenbeschichtung gewünschtenfalls einer Qualitätskontrolle unterzogen werden.

Das beschriebene Verfahren ermöglicht eine dauerhafte Hydrophilierung polymerer Innenoberflächen von Hohlkörpern bzw. Gefäßen, die eine geringe Gerinnungsneigung der dahindurch geleiteten oder der darin temporär aufbewahrten Körperflüssigkeiten oder Zellsuspensionen möglich machen.

Der Fachmann wird erkennen, dass verschiedene Abwandlungen und Ergänzungen an den oben beschriebenen Ausführungsbeispielen möglich sind, ohne den Schutzbereich der beigefügten Ansprüche zu verlassen.

## Patentansprüche

1. Hohlkörper zur temporären Aufnahme von Körperflüssigkeiten, insbesondere Blut, oder Zellsuspensionen,
**gekennzeichnet durch**
eine hydrophilierende Zweischritt-PECVD/CVD-Beschichtung an seiner inneren Oberfläche.

2. Hohlkörper gemäß Anspruch 1, wobei ein Wasserkontaktwinkel der beschichteten inneren Oberfläche unter 2° beträgt.

3. Hohlkörper gemäß Anspruch 2, wobei ein Wasserkontaktwinkel der beschichteten inneren Oberfläche unter 0,5° beträgt.

4. Hohlkörper gemäß einem der Ansprüche 1 bis 3, mit einer polymeren Beschichtung aus Acrylsäure-Monomeren.

5. Hohlkörper gemäß einem der Ansprüche 1 bis 4, mit einem Kunstharz als dem Oberflächenmaterial des Hohlkörpers.

6. Hohlkörper gemäß einem der Ansprüche 1 bis 5, mit genau einer Öffnung.

7. Hohlkörper gemäß Anspruch 6, welcher ein Beutel oder eine Flasche ist.

8. Hohlkörper gemäß einem der Ansprüche 1 bis 5, mit zwei Öffnungen, zwischen denen die Körperflüssigkeit bzw. Zellsuspension bei Benutzung strömt.

9. Hohlkörper gemäß Anspruch 8, welcher ein Rohr oder ein Schlauch ist.

10. Hohlkörper gemäß Anspruch 8, welcher aktivierbarer Teil einer Vorrichtung zur Beaufschlagung der Körperflüssigkeit bzw. Zellsuspension mit einer äußeren Einwirkung ist.

11. Verfahren zur hydrophilierenden Beschichtung eines Hohlkörpers zur temporären Aufnahme einer Körperflüssigkeit oder Zellsuspension an seiner inneren Oberfläche, umfassend:
- Aktivieren der inneren Oberfläche mit einem hauptsächlich aus Inertgas bestehenden Plasma in dem Hohlkörper;
- Vorbeschichten der aktivierten Oberfläche mit einem aus sowohl Inertgas als auch Reaktivgas bestehenden Plasma; und
- Nachbeschichten der so vorbeschichteten Oberfläche mit einer Reaktivgaskomponente ohne Plasmaeinwirkung.

12. Verfahren gemäß Anspruch 11, wobei die Vorbeschichtung weniger als 1 Minute dauert.

13. Verfahren gemäß Anspruch 11 oder 12, wobei die Reaktivgaskomponente Acrylsäure oder deren Anhydrid ist.

14. Verfahren gemäß einem der Ansprüche 11 bis 13, wobei das Oberflächenmaterial des Hohlkörpers ein Kunstharz ist.

15. Verfahren gemäß einem der Ansprüche 11 bis 14, zur Herstellung des Hohlkörpers gemäß einem der Ansprüche 1 bis 10.

## Claims

1. A hollow body for temporarily accommodating a body liquid, in particular blood, or a cell suspension,
**characterized by**
a hydrophilizing two-step PECVD/CVD-coating on its inner surface.

2. The hollow body according to claim 1, wherein a water contact angle of the coated inner surface is below 2°.

3. The hollow body according to claim 2, wherein a water contact angle of the coated inner surface is below 0,5°.

4. The hollow body according to one of claims 1 to 3, with a polymeric coating of acrylic acid monomers.

5. The hollow body according to one of claims 1 to 4, with a plastic resin as the surface material of the hollow body.

6. The hollow body according to one of claims 1 to 5, with exactly one opening.

7. The hollow body according to claim 6, being a bag or a bottle.

8. The hollow body according to one of claims 1 to 5, with two openings, between which the body liquid or cell suspension, respectively, flows in use.

9. The hollow body according to claim 8, being a pipe or a tube.

10. The hollow body according to claim 8, being an actuatable part of a device for applying an external action onto the body liquid or cell suspension, respectively.

11. A process for hydrophilizingly coating a hollow body for temporary accommodation of a body liquid or cell suspension on its inner surface, comprising:
- activating the inner surface with a plasma consisting mainly of an inert gas in the hollow body;
- pre-coating the activated surface with a plasma consisting of both an inert gas and a reactive gas; and
- follow-up-coating the pre-coated surface with a reactive gas component without plasma action.

12. The process according to claim 11, wherein the pre-coating takes less than 1 minute.

13. The process according to claim 11 or 12, wherein the reactive gas component is acrylic acid or the anhydride thereof.

14. The process according to one of claims 11 to 13, wherein the surface material of the hollow body is a plastic resin.

15. The process according to one of claims 11 to 14, for manufacturing the hollow body according to one of claims 1 to 10.

## Revendications

1. Corps creux destiné à recevoir provisoirement des liquides biologiques, en particulier du sang ou des suspensions cellulaires,
**caractérisé par**
un revêtement PECVD/CVD en deux étapes hydrophilisant sur sa surface intérieure.

2. Corps creux selon la revendication 1, un angle de contact de l'eau de la surface intérieure revêtue étant inférieur à 2°.

3. Corps creux selon la revendication 2, un angle de contact de l'eau de la surface intérieure revêtue étant inférieur à 0,5°.

4. Corps creux selon l'une des revendications 1 à 3, comprenant un revêtement polymère de monomères d'acide acrylique.

5. Corps creux selon l'une des revendications 1 à 4, comprenant une résine synthétique comme matériau superficiel du corps creux.

6. Corps creux selon l'une des revendications 1 à 5, comprenant exactement une ouverture.

7. Corps creux selon la revendication 6, lequel est un sachet ou un flacon.

8. Corps creux selon l'une des revendications 1 à 5, comprenant deux ouvertures, entre lesquelles s'écoule le liquide biologique ou la suspension cellulaire lors d'une utilisation.

9. Corps creux selon la revendication 8, lequel est un tube ou un tuyau flexible.

10. Corps creux selon la revendication 8, lequel est une partie activable d'un dispositif destiné à soumettre le liquide biologique ou la suspension cellulaire à un effet extérieur.

11. Procédé de revêtement hydrophilisant d'un corps creux destiné à recevoir provisoirement un liquide biologique ou une suspension cellulaire à sa surface intérieure, comportant :
- l'activation de la surface intérieure comprenant un plasma constitué principalement de gaz inerte dans le corps creux ;
- le revêtement préalable de la surface activée avec un plasma constitué aussi bien de gaz inerte que de gaz réactif ; et
- le revêtement ultérieur de la surface préalablement revêtue avec un composant de gaz réactif sans action plasmatique.

12. Procédé selon la revendication 11, le revêtement préalable durant moins d'une minute.

13. Procédé selon la revendication 11 ou 12, le composant de gaz réactif étant l'acide acrylique ou son anhydride.

14. Procédé selon l'une des revendications 11 à 13, le matériau superficiel du corps creux étant une résine synthétique.

15. Procédé selon l'une des revendications 11 à 14, destiné à la fabrication du corps creux selon l'une des revendications 1 à 10.
